(19) Europäisches Patentamt
European Patent Office
Office européen des brevets

(11) **EP 3 283 993 B1**

(12) **EUROPEAN PATENT SPECIFICATION**

(45) Date of publication and mention
of the grant of the patent:
**15.12.2021 Bulletin 2021/50**

(51) Int Cl.:
*G01G 19/00* (2006.01)    *G16H 20/60* (2018.01)
*G16H 50/20* (2018.01)

(21) Application number: **16780373.3**

(22) Date of filing: **14.04.2016**

(86) International application number:
**PCT/SE2016/000018**

(87) International publication number:
**WO 2016/167701 (20.10.2016 Gazette 2016/42)**

(54) **A PROBABILISTIC CONTEXT FREE GRAMMAR FOR FOOD INTAKE**

PROBABILISTISCHE KONTEXTFREIE GRAMMATIK FÜR NAHRUNGSAUFNAHME

GRAMMAIRE NON CONTEXTUELLE PROBABILISTE POUR LA PRISE DE NOURRITURE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.04.2015 SE 1500181**

(43) Date of publication of application:
**21.02.2018 Bulletin 2018/08**

(73) Proprietor: **Mandometer AB**
**141 04 Huddinge (SE)**

(72) Inventors:
• **BERGH, Cecilia**
**S-118 20 Stockholm (SE)**
• **SÖDERSTEN, Per**
**S-118 20 Stockholm (SE)**
• **IOAKEIMIDIS, Ioannis**
**S-114 17 Stockholm (SE)**
• **PAPAPANAGIOTOU, Vasileios**
**Kavala 65404 (GR)**
• **DIOU, Christos**
**Nea Michaniona 57004 (GR)**
• **DELOPOULOS, Anastasios**
**Panorama 55236 (GR)**

(74) Representative: **Hynell Intellectual Property AB**
**Kabyssgatan 4D**
**120 30 Stockholm (SE)**

(56) References cited:
**WO-A1-2010/070645    WO-A1-2011/037513**
**WO-A1-2011/037513    WO-A1-2013/191613**

US-A1- 2010 236 839    US-A1- 2010 236 839
US-A1- 2014 315 162

• AMFT ET AL: "Probabilistic parsing of dietary
activity events", 4TH INTERNATIONAL
WORKSHOP ON WEARABLE AND
IMPLANTABLE BODY SENSOR NETWORKS
(BSN 2007) : MARCH 26 - 28, 2007; IFMBE
PROCEEDINGS , ISSN 1680-0737 ; VOL. 13,
SPRINGER, DE, vol. 13, 1 January 2007
(2007-01-01), pages 242-247, XP009502266, DOI:
10.1007/978-3-540-70994-7_41 ISBN:
978-3-540-70993-0
• AMFT O ET AL: "On-Body Sensing Solutions for
Automatic Dietary Monitoring", IEEE PERVASIVE
COMPUTING, IEEE SERVICE CENTER, LOS
ALAMITOS, CA, US, vol. 8, no. 2, 1 April 2009
(2009-04-01), pages 62-70, XP011448545, ISSN:
1536-1268, DOI: 10.1109/MPRV.2009.32
• AMFT ET AL.: 'Probabilistic parsing of dietary
activity events' IFMBE PROCEEDINGS - 4TH
INTERNATIONAL WORKSHOP ON WEARABLE
AND IMPLANTABLE BODY SENSOR NETWORKS
vol. 13, 2007, pages 242 - 247, XP009502266
• LOAKIMIDIS ET AL.: 'Description of chewing and
food intake over the course of a meal'
PHYSIOLOGY & BEHAVIOR vol. 104, no. 5, 2011,
pages 761 - 769, XP028390127
• AMFT ET AL.: 'On-Body Sensing Solutions for
Automatic Dietary Monitoring' IEEE PERVASIVE
COMPUTING vol. 8, no. 2, 2009, pages 62 - 70,
XP011448545

**(Cont. next page)**

• PAPAPANAGIOTOU ET AL.: 'Automated Extraction of Food Intake Indicators from Continuous Meal Weight Measurements' ADVANCES IN COMMUNICATION NETWORKING: 20TH EUNICE/IFIP EG 6.2, 6.6 INTERNATIONAL WORKSHOP vol. 9044, no. 558, 15 April 2015, pages 35 - 46, XP047310366

**Description**

**Technical Field**

The present invention pertains to an apparatus comprising a scale connected to a computerized device displaying and depicting a food intake curve for eating, on a screen, of a person eating a measured meal from tableware placed on the scale communicating eating parameters to the computerized device, and a method therefore.

**Background art**

Monitoring and modification of eating behavior through continuous meal weight measurements has been successfully applied in clinical practice to treat obesity and eating disorders. For this purpose, the Mandometer ®, a plate and a scale, along with video recordings of subjects during the course of single meals, has been utilized to assist clinicians in measuring relevant food intake parameters.

Obesity (OB) and Eating Disorders (EDs), including Anorexia Nervosa (AN) and Bulimia Nervosa, affect a great portion of today's modem population. According to the World Health Organisation, more than half a billion people were suffering from OB in 2008[1]. The situation is not predicted to improve in the next years [8]; in fact, the number of people suffering from OB, as well as other diseases which OB is an important contributor for [9], will dramatically increase [8]. Please see the heading "References" [1] to [11] below.

The Mandometer ® treatment method was introduced in 1996 in [2]. The Mandometer ® is a small scale that records the weight of the plate (and food) resting on it at a constant sampling rate; thus producing continuous meal weight recordings. This recording is then processed by clinical experts, sometimes with the help of additional video recordings of the meal, to create the Food Intake (FI) curve of the meal. According to [6], the cumulative FI curve can be sufficiently modelled using a second order polynomial curve $w(t) = \alpha t^2 + \beta t + \gamma$, where $\alpha$ is the FI acceleration, $\beta$ is the initial FI rate and $\gamma = 0$, since no food has been consumed at the beginning of a recording.

The reason for this modelling of the FI curve is based on the evidence presented in [10] and [4], where two main patterns were identified in [10]: linear and decelerated eating. Interestingly, both OB and EDs populations were found to be linear eaters ($\alpha$' 0) in [4], in contrast to normal populations who are characterised by decelerating eating ($\alpha$ < 0). This is evidence that the quadratic modelling of the eating pattern can be used to detect high risk OB and EDs individuals. Furthermore, the use of Mandometer ® can modify the eating pattern of linear eaters, as shown in [11], and thus reduce the risk for developing such diseases. Indeed, in [1], results from 1,428 subjects taken over 18 years show remission rates of 74%, out of which only 10% has relapsed.

However, the processing of the Mandometer® recording is not a trivial task.

Usually, clinicians who process the recordings in order to create the FI curves rely heavily on empirical rules and some guessing, introducing errors in the interpretation. On the other hand, additional video recordings can be used to aid the clinicians, but this process is very laborious and requires additional work (such as synchronisation, etc.).

An apparatus named " Mandometer®" has been developed at the of Applied Neuroendocrinology and Mandometer® Clinic, Karolinska Institutet, Stockholm, Sweden. It consists of a scale that is connected to a computer. A plate is placed on the scale, the patient puts a measured portion of food determined by a therapist on the plate and the computer records and stores the weight loss from the plate while the patient eats.

This yields a curve of eating rate which is visible to the patient on the computer screen during a meal and can be compared to a pre-set eating curve on screen. At regular intervals, a rating scale appears on the monitor of the computer and the patient rates her/his level of fullness/satiety. The scale has numerical values from O (no satiety) to 100 (maximum satiety). As the patients rate their satiety a dot appears on the screen and yields a curve of the development of satiety (fullness). The patients can thus compare their development of fullness to a "normal" fullness curve again pre-set on screen. During "Mandometer®-training" the patient gradually adopts a more normal pattern of eating and satiety by following the training curves, which are displayed on the monitor during the meal. These methods were originally developed for treating eating disorders such as anorexia and bulimia nervosa: they have been evaluated in a randomized controlled trial with an estimated rate of remission of 75%. It was suggested many years ago that obese people eat at an increased rate and in a pilot study on obese adolescents using Mandometer® this observation was confirmed.

WO2011037513 discloses a medical apparatus made up of at least two devices adapted to measure eating rate utilized to teach persons to eat according to a predetermined graphic eating curve displayed on a screen, and a method therefore. There are means in a memory automatically calculating equations for predetermined eating curves based on data obtained from a cumulative food intake in a control meal through an introductory curve followed by training curves. The cumulative food intake is fitted to a quadratic equation $y=ax^2+bx+c$, where the parameter y represents food intake, and represents a change in the slope of the curve over time x, b represents a constant slope of the curve over time, the initial rate of eating, and c represents food intake at the start of a meal, whereby the introductory curve has the same properties as the cumulative food intake obtained in a control meal.

US2010236839 discloses a portable medical apparatus made up of at least two devices adapted to measure eating rate utilized to teach persons to eat according to a predetermined graphic eating curve displayed on a screen and a method therefore. In a specific mode the predetermined graphic curve describes a non-linear descending rate of eating behaviour. The apparatus comprises a device with cellular capabilities, and a scale, which devices communicate through near field radiation such as Bluetooth or IR.

## Summary of the invention

In the present invention a novel algorithm is presented, which automatically constructs the FI curve using only the Mandometer® recording, based on a parametric Probabilistic Context-Free Grammar (PCFG). This algorithm significantly improves over the algorithms presented in [7]. Experiments on a large dataset validate the algorithm's efficiency.

Hence, one aim of the present invention is to set forth an apparatus comprising a scale connected to a computerized device displaying and depicting a food intake curve for eating, on a screen, of a person eating a measured meal from tableware placed on the scale communicating eating parameters to the computerized device. Hereby it comprises:

the curve is derived from a parametric probabilistic context free grammar algorithm for food intake analysis on continuous meal weight measurements, through the following processing, conducted in five calculation steps a.) to e.), by the computerized device:

a) a pre-processing stage comprising of an opening morphological operator with adaptive structure element size selected using delta-coefficients;

b.) definition of the recorded weight at each time as $w(n)$ and definition of the parameters: $d_w(n)=w(n)-w(n-1)$ and

$$x(n) = \begin{cases} d \text{ if } d_w(n) < 0 \\ r \text{ if } d_w(n) > 0 \\ e \text{ if } d_w(n) = 0 \end{cases};$$

c.) definition of the Context-free grammar:

$$S \rightarrow BS\,|FS\,|AS\,|e \quad (1)$$
$$B \rightarrow D\,|RD \quad (2)$$
$$F \rightarrow R \quad (3)$$
$$A \rightarrow RSD \quad (4)$$
$$R \rightarrow rr^* \quad (5)$$
$$D \rightarrow dd^* \quad (6)$$

where * is the Kleene star operator, and symbols r and d are the PCFG terminal symbols;

d.) Definition of the probability for the substitution rules (2), (3) or (4), corresponding to Bite, Food Addition or Artefact events all other rules have probability of 1; and

e.) Selection of the parse tree with the maximum likelihood for the observed food intake measurements, by utilizing the symbols B (bites), F (food additions) and A (artefacts) to interpret the events of the curve and reconstruct a corrected food intake curve.

Moreover, the present invention sets forth a method adapted to an apparatus comprising a scale connected to a computerized device displaying and depicting a food intake curve for eating, on a screen, of a person eating a measured meal from tableware placed on the scale communicating eating parameters to the computerized device. Hereby the method comprises the steps of:

deriving the curve from a parametric probabilistic context free grammar algorithm for food intake analysis on continuous meal weight measurements, through the following processing, conducted in five calculation steps a.) to e.), by the computerized device:

a) a pre-processing stage comprising of an opening morphological operator with adaptive structure element size selected using delta-coefficients;

b.) definition of the recorded weight at each time as $w(n)$ and definition of the parameters: $d_w(n) = w(n) - w(n-1)$ and

$$x(n) = \begin{cases} d \text{ if } d_w(n) < 0 \\ r \text{ if } d_w(n) > 0 \\ e \text{ if } d_w(n) = 0 \end{cases};$$

c.) definition of the Context-free grammar:

$$S \rightarrow BS\,|FS\,|AS\,|e \quad (1)$$
$$B \rightarrow D\,|RD \quad (2)$$
$$F \rightarrow R \quad (3)$$
$$A \rightarrow RSD \quad (4)$$
$$R \rightarrow rr^* \quad (5)$$
$$D \rightarrow dd^* \quad (6)$$

where is the Kleene star operator, and symbols r and d are the PCFG terminal symbols;

d.) defining the probability for the substitution rules (2), (3) or (4), corresponding to Bite, Food Addition or Artefact events all other rules have probability of 1; and

e.) selecting the parse tree with the maximum likelihood for the observed food intake measurements, by utilizing the symbols B (bites), F (food additions) and A (artefacts) to interpret the events of the curve and reconstruct a corrected food intake curve.

**Brief description of the drawings**

Henceforth, reference is had to the attached drawings for a better understanding of the present invention, and it's given examples and embodiments, wherein:

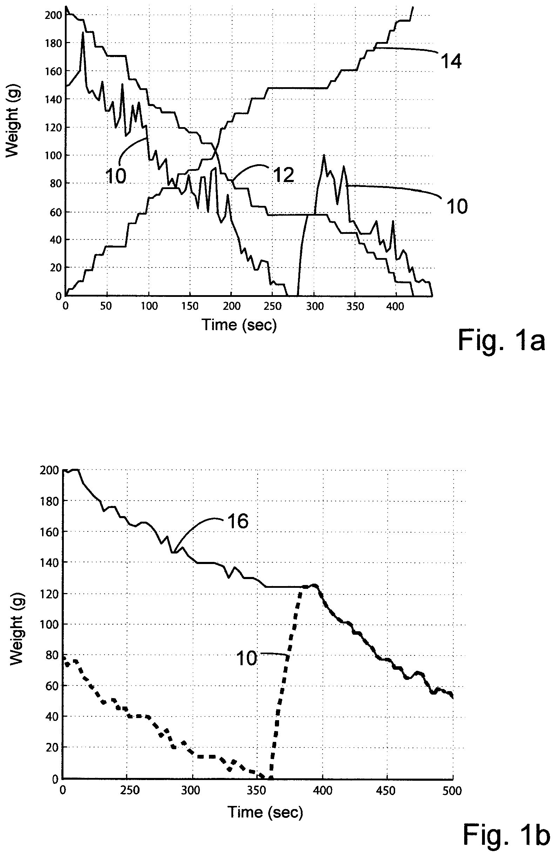

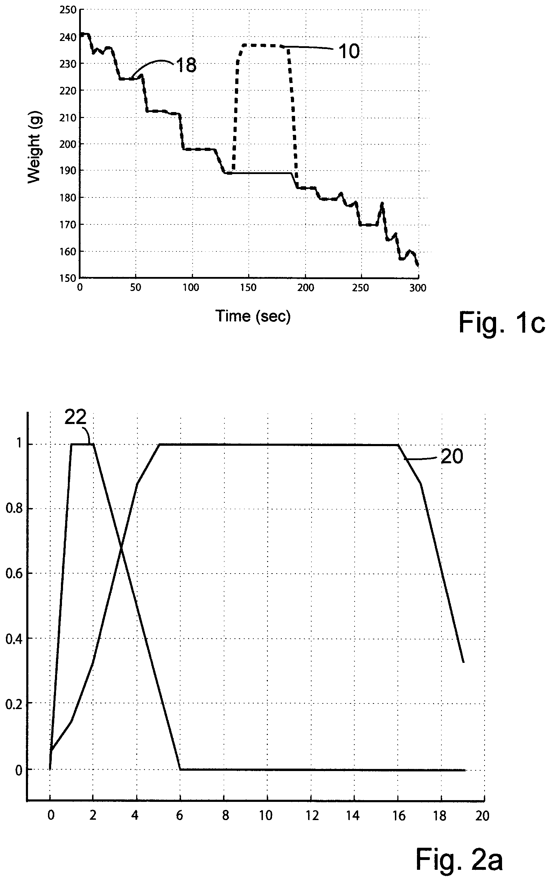

**Fig. 1a, b, and c** illustrate the processing of the Mandometer ® recording in accordance with the present invention;
**Fig. 1a** depicts the raw recording, the processed recording, and the FI curve;
**Fig. 1b and 1c** depicts the processing of an FA and an artefact; and
**Fig. 2a, b, and c** depict probability density functions for the parameters of PCFG events in accordance with the present invention.

**Tables**

The tables of the present invention are depicted at the end of the present description, wherein:

**Table I** depicts the length of structuring element u used in pre-processing based on the value of parameter $\xi$;
**Table II** depicts the four classes for the $\alpha$ coefficient of the quadratic model of a FI curve;
**Table III** depicts the five classes for the $\beta$ coefficient of the quadratic model of an FI curve;
**Table IV** depicts a confusion matrix for coefficient $\alpha$ (eating pattern). Overall accuracy is 90.35%;
**Table V** depicts a confusion matrix for coefficient $\beta$. Overall accuracy is 87.72%s; and
Table VI depicts MAD and StdAD of indicators.

**References**

[1] Cecilia Bergh, Monica Callmar, Sophia Danemar, Mats Holcke, Su-¨ sanne Isberg, Michael Leon, Jessica Lindgren, Asa Lundqvist, Maria° Niinimaa, Barbro Olofsson, et al. Effective treatment of eating disor-~ ders: Results at multiple sites. Behavioral neuroscience, 127(6):878, 2013.
[2] Cecilia Bergh, Stale Eklund, Mats Eriksson, Greger Lindberg, and° Per Sodersten.¨ A new treatment of anorexia nervosa. The Lancet, 348(9027):611-612, 1996.
[3] Rafael C Gonzalez and Richard E Woods. Digital image processing, 2002.
[4] Ioannis Ioakimidis, Modjtaba Zandian, Cecilia Bergh, and Per Sodersten. A method for the control of eating rate:

a potential interven-¨ tion in eating disorders. Behavior research methods, 41(3):755—760, 2009.

[5] Ioannis Ioakimidis, Modjtaba Zandian, Florian Ulbl, Carina Alund,° Cecilia Bergh, and Per Sodersten. Food intake and chewing in women.¨ Neurocomputing, 84:31-38, 2012.

[6] Harry R Kissileff, John Thornton, and Emil Becker. A quadratic equation adequately describes the cumulative food intake curve in man. Appetite, 3(3):255-272, 1982.

[7] Vasileios Papapanagiotou, Christos Diou, Billy Langlet, Ioannis Ioakimidis, and Anastasios Delopoulos. Automated extraction of food intake indicators from continuous weight measurements. aaa, 1:1-2, 2015.

[8] Laura Webber, Diana Divajeva, Tim Marsh, Klim McPherson, Martin Brown, Gauden Galea, and Joao Breda. The future burden of obesityrelated diseases in the 53 who european-region countries and the impact of effective interventions: a modelling study. BMJ open, 4(7):e004787, 2014.

[9] Ram Weiss and Sonia Caprio. The metabolic consequences of childhood obesity. Best Practice & Research Clinical Endocrinology & Metabolism, 19(3):405-419, 2005.

[10] Modjtaba Zandian, Ioannis Ioakimidis, Cecilia Bergh, Ulf Brodin, and Per Sodersten. Decelerated and linear eaters: effect of eating rate on¨ food intake and satiety. Physiology & behavior, 96(2):270-275, 2009.

[11] Modjtaba Zandian, Ioannis Ioakimidis, Cecilia Bergh, and Per Sodersten. Linear eaters turned decelerated: Reduction of a risk for¨ disordered eating? Physiology & behavior, 96(4):518-521, 2009.

**Detailed description of preferred embodiments**

The present invention regards monitoring and modification of eating behavior through continuous meal weight measurements, and has been successfully applied in clinical practice to treat obesity and eating disorders. For this purpose, the Mandometer ®, a plate scale, along with video recordings of subjects during the course of single meals, has been used to assist clinicians in measuring relevant food intake parameters. In this work, a novel algorithm for automatically constructing a subject's food intake curve is presented utilizing only the Mandometer ® weight measurements. This eliminates the need for direct clinical observation or video recordings, thus significantly reducing the manual effort required for analysis. The proposed algorithm aims at identifying specific meal related events (e.g. bites, food additions, artifacts), by applying an adaptive pre-processing stage using Delta coefficients, followed by event detection based on a parametric Probabilistic Context-Free Grammar on the derivative of the recorded sequence. Experimental results on a dataset of 114 meals from individuals suffering from obesity or eating disorders, as well as from individuals with normal BMI, demonstrate the effectiveness of the proposed approach.

The present invention apparatus consist of a scale communicating wireless/cable with a computerized apparatus with a screen for instance a smartphone, tablet, lap-top, or the like. A plate is placed on the scale with food, and a person is eating from the plate, and every bite is registered and communicated. The computerized device displays the actual eating curve on the screen from a person eating in real time. This curve is compared to a curve made up by measurements from persons that have a healthy eating behavior. Through this proceeding persons that have an unhealthy eating behavior can practice with the computerized apparatus to achieve a healthy eating behavior. The present invention sets forth an eating curve for a person eating in real time, which automatically presents the curve on the screen through an algorithm by considering/predicting events that occur through the eating, such events could be the pressure by a fork on the plate, dropping food on the table, which provides a false result for a bite during a meal. Other parameters are also considered, and discussed in the following description.

In this work a novel algorithm is presented in accordance with the present invention, which automatically constructs the FI curve using only the Mandometer ® recording, based on a parametric Probabilistic Context-Free Grammar (PCFG). This algorithm significantly improves over the algorithms presented in [7]. Experiments on a large dataset validate the algorithm's efficiency.

The algorithm aims at constructing the (cumulative) FI curve of the meal. The FI curve describes the weight of food the subject has consumed throughout the duration of the meal. For this purpose, the raw meal recording is processed, yielding a version free of non-eating-related weight fluctuations seen in Fig. 1a. These fluctuations can either be Food Additions (FAs) or artefacts. FA refers to adding additional quantity of food during the meal evolution seen in Fig. 1b. On the other hand, an artefact describes a temporary shifting of the weight, first a rise and then a drop; this can have various causes, such as pressure applied by a knife, which registers a spike-like artefact, or the resting of a fork/spoon on the plate, which registers a plateau-like shape, Fig. 1c. As a result, the PCFG assumes the following symbols: $B$ for a bite event, $F$ for a FA event, and A for an artefact event. Each event is assigned a probability based on parameters extracted from the meal recording. Finally, the interpretation of the meal with the highest likelihood is obtained.

Fig. 1 Illustrates the processing of the Mandometer ® recording. Fig. 1a shows the raw recording 10, the processed recording 12, and the FI curve 14, and Fig. 1b and 1c show the processing of an FA 16, 18 and an artefact 10.

Before applying the PCFG, some pre-processing is performed on the raw recorded data. Initially, the start and end of the meal are first detected. Given a sequence of recorded meal weights $w'_0(n)$, the start and end of the meal are considered as the first and last sample of the recording at which the recording is decreasing; all samples not in-between the first and last one are discarded. The resulting sequence is denoted as $w_0(n)$.

A smoothing step is then applied, that removes spikes caused during fork/knife use on the plate. The smoothing is performed by applying the *opening* morphological operator [3] on the raw data, using a structuring element $u$ of ones. The length of the structuring element is determined automatically, based on the raw data with the help of the Delta coefficients (or Deltas).

The Deltas capture the trend (increasing or decreasing) of the curve. Given a sequence $x(n)$, they are defined as

$$\delta_D(n) = \frac{\sum_{k=-D}^{D} k x(n+k)}{\sum_{k=-D}^{D} k^2} \qquad (1)$$

where the parameter $D$ defines the range $[-D,D]$ where the trend of the curve is estimated.

Given a meal recording $w_0(n)$ of $N$ samples, the length of the structuring element u is determined based on the following quantity

$$\xi = \frac{\sum_{k=1}^{N} |\delta_5(w_0(k)) - \delta_{15}(w_0 1(k))|}{\sum_{k=1}^{N} |\delta_{15}(w_0(k))|} \qquad (2)$$

This is a rough estimation of the smoothness of the curve; the higher the value of $\xi$ the less smooth the meal is, and thus harder smoothing is required. The length of $u$ is determined using Table I. The result of the application of *opening* on $w_0(n)$ using $u$ is denoted as $w(n)$.

The generative model for the PCFG, as described in the first paragraphs of Section II, is expressed by the following substitution rules

$$S \rightarrow BS\,|FS\,|AS\,|e \qquad (3)$$
$$B \rightarrow D\,|RD \qquad (4)$$
$$F \rightarrow R \qquad (5)$$
$$A \rightarrow RSD \qquad (6)$$

where $S$ is the starting symbol, e is the empty string symbol, and "I" denotes the *or* operator. Symbols $R$ and $D$ describe weight increasing (rise) and decreasing (drop) segments, and substitute the following strings

$$R \rightarrow rr^* \qquad (7)$$
$$D \rightarrow dd^* \qquad (8)$$

where $^*$ is the Kleene star operator, and symbols $r$ and $d$ are the PCFG terminal symbols. They are obtained directly from the derivative of the meal recording, forming the string representation x of the meal using the following equation

$$x(n) = \begin{cases} d, & \text{if } d_w(n) < 0 \\ r, & \text{if } d_w(n) > 0 \\ e, & \text{if } d_w(n) = 0 \end{cases} \qquad (9)$$

The derivative of $w(n)$ is computed as $d_w(n) = w(n) - w(n-1)$. Thus, each sample of the recording produces one terminal symbol, with the exception of the first sample.

The probabilities assigned to substitution rules of Eq. 7 and Eq. 8 are $p(R) = 1$ and $P(D) = 1$. This choice is based on the fact that symbols have no physical interpretation; they are merely means to aggregate subsequent rises or drops, in order to remove the dependence on the sampling rate of the Mandometer ®.

On the other hand, a bite event registers a drop on the recording. Usually however, the applied force by a fork/knife causes a temporary weight rise; this pattern is captured by the rule of Eq. 4. Total weight drop during a bite event, denoted $B_{dw}$, is usually 5 to 15 grams, and total duration $B_{dt}$ is approximately 1 to 3 samples. These two parameters are independent, and thus the probability of a segment of the recording being a bite event B is $p(B) = p_{B1}(B_{dw})p_{B2}(B_{dt})$.

The PDFs $p_{B1}$ 20 and $p_{B2}$ 22 are shown in Fig. 2a.

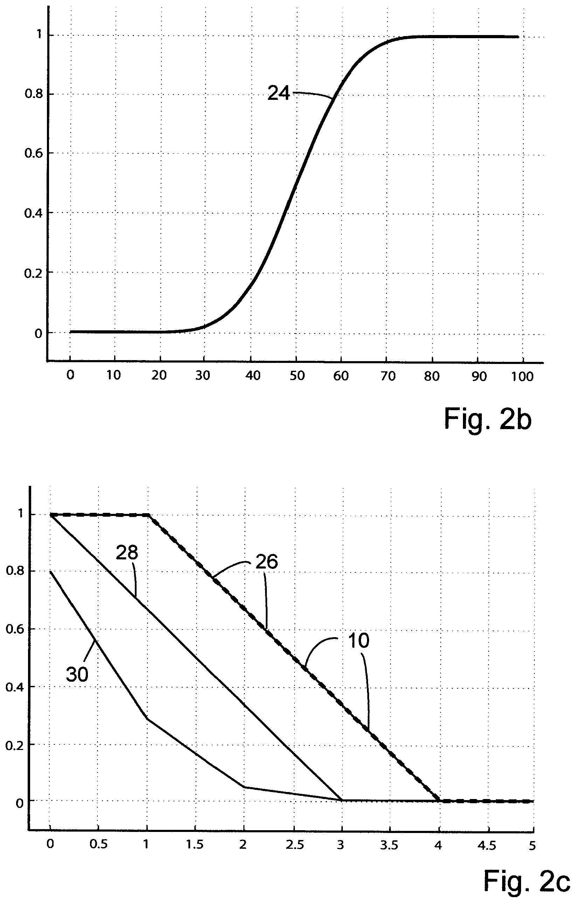

Similarly, a FA event registers a weight rise (see Fig. 1b), which is captured by Eq. 5. Therefore, the probability of a segment being a FA event $F$ is $p(F) = p_F(F_{dw})$, where the PDF $p_F$ 24 is shown in Fig. 2b, and $F_{dw}$ is the weight increase during the FA.

The artefact event describes the phenomenon during which (a) extra weight is added on the plate e.g. a spoon or a knife, not food however, (b) optionally something else might occur e.g. a bite, and (c) the weight is removed from the plate. This is essentially captured by Eq. 6. The probability of such an event is based on four parameters: (a-b) the durations of $R$ and $D$, denoted $R_{dt}$ and $D_{dt}$ respectively, which must be short, (c) the difference between the weight increase during $R$ and the weight decrease during $D$, $A_{dw}$, which must be close to zero, and (d) the duration of intermediate event $S$, $S_{dt}$, which should also not be too long. As a result, the probability of an event $A$ is $p(A) = pA1$ $(Rdt)pA2(Ddt)pA3(Adw)pA4(Sdt)$, where $pAi$, $i$ = 1,2,3,4, are shown in in Fig. 2c, wherein the probability curves are designated as $pA1$ 26, $pA2$ 10, $pA3$ 30, and $pA4$ 28. Note that curve 26 is a straight line following the curve 10 depicted as a broken line.

The probability of substituting $S$ using any of the four rules of Eq. 3 depends on the particular substitution only. No other assumptions are made about the events, such as the total number of FAs, etc. Thus, $p(S \rightarrow XS) = p(X)p(S)$ for $X = B,F,A$, and $p(S \rightarrow e) = 1$.

The PCFG is ambiguous: more than one parse trees exist for each input string. Thus, dynamic programming is employed in order to determine all possible interpretations. For each tree, a likelihood is computed that the tree correctly interprets the meal. As a result, the parse tree with the highest likelihood is selected as the final interpretation of events during the recorded meal.

Once the final interpretation of a meal has been obtained, the FI intake curve is reconstructed based on the smoothed recording $w(n)$ and the PCFG events. FAs are removed, by adjusting the recording as if the entire food quantity had been added on the plate before the beginning of the meal (see Fig. 1b). Furthermore, artifacts are also removed, by cancelling out the weight rise and drop of the $R$ and $D$ parts of the event, and by subtracting the weight offset caused by the $R$ and $D$ from all samples of the embedded $S$ part, if it exists (see Fig. 1c).

Finally, each bite is adjusted so that the entire weight drop occurs at the last sample of the bite. Finally, the PCFG FI curve $y(n)$ is obtained by subtracting the value of the last sample from all samples, so that zero left-overs occur, and then flipping the curve upside-down, or simply y(n) = wc(1) - wc(n).

In order to evaluate the effectiveness of the proposed algorithm, a dataset consisting of 114 meal recordings is used. These have been recorded from 105 females and 9 males, out of which 49 have been characterised as normalweight, with mean age of 22.8 years and mean Body-Mass Index (BMI) of $22.2 kg/m^2$, 23 have been characterised as obese, with mean age of 35.22 years and mean BMI of 37.21 $kg/m^2$, and the rest 46 as AN cases, with mean age of 21.7 years and mean BMI of $17.4 kg/m^2$.

For each meal, both the raw Mandometer ® recordings, as well as the ground truth FI curves are provided. The ground truth FI curves of the clinical recordings have been produced by clinical experts at Mando ® clinics and researchers at Karolinska Institutet, based on both the raw Mandometer ® data and video recordings of subjects eating. The standard procedure for this process is described in [5].

To evaluate the effectiveness of the PCFG algorithm, we apply it on each raw meal recording and obtain the PCFG FI curve. For each meal, we extract behavioural indicators both from the ground truth FI curve and the PCFG FI curve for evaluation. These indicators include: coefficients $\alpha$ and $\beta$ of the quadratic approximation of the FI curve, total FI weight (in grams), total meal duration (in seconds), and average bite size (also in grams). For coefficients $\alpha$ and $\beta$, four and five classes are identified respectively by clinical experts, and are presented in Tables II and III.

Results for the $\alpha$ and $\beta$ coefficients are presented in confusion matrices, where the ground truth class is assigned based on the value of the coefficient extracted from the ground truth FI curve, and the predicted class based on the value of the coefficient extracted from the PCFG FI curve. For the remaining indicators, the Mean Absolute Difference (MAD) and the Standard Deviation of Absolute Difference (StdAD) are computed across all meals.

The Fig. 2 depicts probability density functions for the parameters of the PCFG events. They have been determined based on statistical analysis of the available recordings and their ground truth.

The experimental results are presented in Tables IV, V and VI. Table IV presents the confusion matrix for the classification task of the eating pattern, based on the value of coefficient $\alpha$. Detection accuracy is 90%, which is significantly higher than the top accuracy of 83% reported for the algorithms in [7]. Table V presents the confusion matrix for the classification task of coefficient $\beta$; detection accuracy is 88%.

Table VI presents the MAD and StdAD across all meals, for each of the remaining three indicators: total FI in grams, meal duration in seconds, and average bite size in grams.

These results indicate that the effectiveness of the PCFG algorithm is very high. Confusion in the eating pattern is minimal, and for most misclassified meals the predicted class is adjacent to the actual, indicating a relatively small error of the actual value of the $\alpha$ coefficient. The same is true for the classification based on the $\beta$ coefficient. Finally, the effectiveness of the algorithm on the rest of the indicators is also high, as indicated be the MAD of total FI which is only 10 grams (it was 26 grams for the best case in [7]), and the MAD of the average bite size which is less than 1 gram.

The Mandometer ® treatment is one of the most effective methods against OB and EDs. It is based on analysing continuous meal weight recordings and constructing the FI curve of the meal, and has been shown to achieve very high remission rates, and very low relapse rates as well. In this work we have presented an algorithm that automatically constructs the FI curve based solely on the Mandometer ® recordings. The effectiveness of the algorithm has been demonstrated on a large dataset that includes normal, OB and AN cases of varying BMI. MAD of the average bite size is less than 1 gram.

**Tables**

TABLE I: Length of structuring element u used in pre-processing based on the value of parameter $\xi$.

| $\xi$ | | < 0.3 | < 0.6 | < 1 | < 2 | < 3 | < + inf |
|---|---|---|---|---|---|---|
| *length(u)* | | 2 | 3 | 5 | 7 | 11 | 15 |

TABLE II: The four classes of α coefficient of the quadratic model of a FI curve.

| Class | Range ($\cdot 10^{-3}$) |
|---|---|
| $C_1^\alpha$ (decelerated) | $(-\infty, -4]$ |
| $C^\alpha$ (semi-decelerated) | $(-4, -1.5]$ |
| $C_3^\alpha$ (linear) | $(-1.5, 1.5]$ |
| $C_4^\alpha$ (accelerated) | $(1.5, +\infty)$ |

TABLE III: depicts the five classes for the $\beta$ coefficient of the quadratic model of a FI curve.

| $C_1^\beta$ | $C_2^\beta$ | $C_3^\beta$ | $C_4^\beta$ | $C_5^\beta$ |
|---|---|---|---|---|
| $(-\infty, -2]$ | $(-2, -1.5]$ | $(-1.5, -1]$ | $(-1, -0.5]$ | $(0.5, +\infty)$ |

TABLE IV is a confusion matrix for coefficient $\alpha$ (eating pattern). Overall accuracy is 90.35%.

|  |  | Predicted | | | |
|---|---|---|---|---|---|
|  |  | $C_1^{\alpha}$ | $C_2^{\alpha}$ | $C_3^{\alpha}$ | $C_4^{\alpha}$ |
| Actual | $C_1^{\alpha}$ | 3 | 0 | 0 | 0 |
|  | $C_2^{\alpha}$ | 0 | 5 | 1 | 0 |
|  | $C_3^{\alpha}$ | 0 | 1 | 57 | 6 |
|  | $C_4^{\alpha}$ | 0 | 1 | 2 | 38 |

TABELL V: is a confusion matrix for coefficient $\beta$. Overall accuracy is 87.72%.

|  |  | Predicted | | | | |
|---|---|---|---|---|---|---|
|  |  | $C_1^{\beta}$ | $C_2^{\beta}$ | $C_3^{\beta}$ | $C_4^{\beta}$ | $C_5^{\beta}$ |
| Actual | $C_1^{\beta}$ | 0 | 0 | 0 | 0 | 0 |
|  | $C_2^{\beta}$ | 1 | 11 | 2 | 1 | 0 |
|  | $C_3^{\beta}$ | 0 | 5 | 35 | 3 | 0 |
|  | $C_4^{\beta}$ | 0 | 0 | 2 | 53 | 0 |
|  | $C_5^{\beta}$ | 0 | 0 | 0 | 0 | 1 |

TABELL VI: depicts the MAD and StdAD of indicators.

| Indicator | MAD StdAD |
|---|---|
| Total food intake (grams) | 10.00 30.74 |
| Duration (seconds) | 16.49 30.84 |
| Average bite size (grams) | 0.61 1.37 |

## Claims

1. An apparatus comprising a scale and a computerized device connected to the scale, the apparatus configured for displaying and depicting a food intake curve for eating, on a screen, of a person eating a measured meal from tableware placed on the scale communicating eating parameters to the computerized device, **characterized in that**: the computerized device is configured for deriving the food intake curve from a parametric probabilistic context free grammar algorithm for food intake analysis on only continuous meal weight measurements, through the following processing, conducted in five calculation steps a.) to e.), by the computerized device:

   a) a pre-processing stage comprising of an opening morphological operator with adaptive structure element size selected using delta-coefficients;
   b.) definition of the recorded weight at each time as $w(n)$ and definition of the parameters: $d_w(n) = w(n) - w(n$

$$x(n) = \begin{cases} d \text{ if } d_w(n) < 0 \\ r \text{ if } d_w(n) > 0 \\ e \text{ if } d_w(n) = 0 \end{cases}$$

   — 1) and
   c.) definition of the Context-free grammar:

$$S \rightarrow BS \,|FS\, |AS\, |e \quad (1)$$
$$B \rightarrow D\, |RD \quad (2)$$
$$F \rightarrow R \quad (3)$$
$$A \rightarrow RSD \quad (4)$$
$$R \rightarrow rr^* \quad (5)$$

(continued)

$$D \to dd^*  \quad (6)$$

where $^*$ is the Kleene star operator, and symbols r and d are the PCFG terminal symbols,

and wherein e is the empty string symbol, symbols R and D describe weight increasing and decreasing segments and x is the string representation of the meal;

d.) Definition of the probability for the substitution rules (2), (3) or (4), corresponding to Bite, Food Addition or Artefact events all other rules have probability of 1; and

e.) Selection of the parse tree with the maximum likelihood for the observed food intake measurements, by utilizing the symbols B (bites), F (food additions) and A (artefacts) to interpret the events of the curve and reconstruct a corrected food intake curve.

2. A method used with an apparatus comprising a scale and a computerized device connected to the scale, the method consisting of displaying and depicting a food intake curve for eating, on a screen, of a person eating a measured meal from tableware placed on the scale communicating eating parameters to the computerized device, **characterized in that**:

the food intake curve is derived from a parametric probabilistic context free grammar algorithm for food intake analysis on only continuous meal weight measurements, through the following processing, conducted in five calculation steps a.) to e.), by the computerized device:

a) a pre-processing stage comprising of an opening morphological operator with adaptive structure element size selected using delta-coefficients;

b.) definition of the recorded weight at each time as $w(n)$ and definition of the parameters: $d_w(n) = w(n) - w(n$

$$x(n) = \begin{cases} d \text{ if } d_w(n) < 0 \\ r \text{ if } d_w(n) > 0 \\ e \text{ if } d_w(n) = 0 \end{cases} $$

$-1)$ and

c.) definition of the Context-free grammar:

$$S \to BS\,|FS\,|AS\,|e \quad (1)$$
$$B \to D\,|RD \quad (2)$$
$$F \to R \quad (3)$$
$$A \to RSD \quad (4)$$
$$R \to rr^* \quad (5)$$
$$D \to dd^* \quad (6)$$

where $^*$ is the Kleene star operator, and symbols r and d are the PCFG terminal symbols, and wherein e is the empty string symbol, symbols R and D describe weight increasing and decreasing segments and x is the string representation of the meal;

d.) defining the probability for the substitution rules (2), (3) or (4), corresponding to Bite, Food Addition or Artefact events all other rules have probability of 1; and

e.) selecting the parse tree with the maximum likelihood for the observed food intake measurements, by utilizing the symbols B (bites), F (food additions) and A (artefacts) to interpret the events of the curve and reconstruct a corrected food intake curve.

**Patentansprüche**

1. Vorrichtung, die eine Waage und eine mit der Waage verbundene computergestützte Vorrichtung umfasst, wobei die Vorrichtung zur Anzeige und Darstellung einer Nahrungsaufnahmekurve für das Essen einer Person, die eine abgemessene Mahlzeit von einem auf der Waage platzierten Geschirr isst, auf einem Bildschirm und zur Übermittlung von Essparametern an die computergestützte Vorrichtung ausgebildet ist, **dadurch gekennzeichnet, dass:**
die computergestützte Vorrichtung zur Ableitung der Nahrungsaufnahmekurve aus einem parametrischen probabilistischen kontextfreien Grammatikalgorithmus für die Analyse der Nahrungsaufnahme auf nur kontinuierlichen Mahlzeitengewichtsmessungen durch die folgende, in fünf Berechnungsschritten a.) bis e.) durchgeführte Verarbeitung durch die computergestützte Vorrichtung ausgebildet ist:

a) eine Vorverarbeitungsstufe, bestehend aus einem morphologischen Öffnungsoperator mit adaptiver Strukturelementgröße, die mit Hilfe von Delta-Koeffizienten ausgewählt wird;

b.) Definition des aufgezeichneten Gewichts zu jedem Zeitpunkt als $w(n)$ und Definition der Parameter:

$$d_w(n) = w(n) - w(n-1) \quad \text{und} \quad x(n) = \begin{cases} d \text{ wenn } d_w(n) < 0 \\ r \text{ wenn } d_w(n) > 0 \\ e \text{ wenn } d_w(n) = 0 \end{cases} ;$$

c.) Definition der kontextfreien Grammatik:

$$\begin{aligned}
S &\to BS\,|FS\,|AS\,|e & (1) \\
B &\to D\,|RD & (2) \\
F &\to R & (3) \\
A &\to RSD & (4) \\
R &\to rr^* & (5) \\
D &\to dd^* & (6)
\end{aligned}$$

wobei * der Kleene-Stern-Operator ist,
und die Symbole r und d die PCFG-Terminalsymbole sind;

und wobei e das leere Zeichenketten-Symbol ist, die Symbole R und D gewichtszunehmende und gewichtsabnehmende Segmente beschreiben und x die Zeichenketten-Darstellung der Mahlzeit ist;
d.) Definition der Wahrscheinlichkeit für die Substitutionsregeln (2), (3) oder (4), die den Ereignissen "Bissen", "Nahrungsergänzung" oder "Artefakt" entsprechen, alle anderen Regeln haben eine Wahrscheinlichkeit von 1; und
e.) Auswahl des Parse-Baums mit der größten Wahrscheinlichkeit für die beobachteten Messungen der Nahrungsaufnahme, indem die Symbole B (Bisse), F (Nahrungsergänzungen) und A (Artefakte) verwendet werden, um die Ereignisse der Kurve zu interpretieren und eine korrigierte Kurve der Nahrungsaufnahme zu rekonstruieren.

**2.** Verfahren, das mit einer Vorrichtung verwendet wird, die eine Waage und eine mit der Waage verbundene computergestützte Vorrichtung umfasst, wobei das Verfahren in der Anzeige und Darstellung einer Nahrungsaufnahmekurve für das Essen einer Person, die eine abgemessene Mahlzeit von einem auf der Waage platzierten Geschirr isst, auf einem Bildschirm und in der Übermittlung von Essparametern an die computergestützte Vorrichtung besteht, **dadurch gekennzeichnet, dass:**
die Nahrungsaufnahmekurve aus einem parametrischen probabilistischen kontextfreien Grammatikalgorithmus für die Analyse der Nahrungsaufnahme auf nur kontinuierlichen Mahlzeitengewichtsmessungen durch die folgende, in fünf Berechnungsschritten a.) bis e.) durchgeführte Verarbeitung durch die computergestützte Vorrichtung abgeleitet ist:

a) eine Vorverarbeitungsstufe, bestehend aus einem morphologischen Öffnungsoperator mit adaptiver Strukturelementgröße, die mit Hilfe von Delta-Koeffizienten ausgewählt wird;
b.) Definition des aufgezeichneten Gewichts zu jedem Zeitpunkt als $w(n)$ und Definition der Parameter:

$$d_w(n) = w(n) - w(n-1) \quad \text{und} \quad x(n) = \begin{cases} d \text{ wenn } d_w(n) < 0 \\ r \text{ wenn } d_w(n) > 0 \\ e \text{ wenn } d_w(n) = 0 \end{cases} ;$$

c.) Definition der kontextfreien Grammatik:

$$S \rightarrow BS|FS|AS|e \quad (1)$$
$$B \rightarrow D|RD \quad (2)$$
$$F \rightarrow R \quad (3)$$
$$A \rightarrow RSD \quad (4)$$
$$R \rightarrow rr^* \quad (5)$$
$$D \rightarrow dd^* \quad (6)$$

wobei * der Kleene-Stern-Operator ist,
und die Symbole r und d die PCFG-Terminalsymbole sind;

und wobei e das leere Zeichenketten-Symbol ist, die Symbole R und D gewichtszunehmende und gewichtsabnehmende Segmente beschreiben und x die Zeichenketten-Darstellung der Mahlzeit ist;
d.) Definieren der Wahrscheinlichkeit für die Substitutionsregeln (2), (3) oder (4), die den Ereignissen "Bissen", "Nahrungsergänzung" oder "Artefakt" entsprechen, alle anderen Regeln haben eine Wahrscheinlichkeit von 1; und
e.) Auswählen des Parse-Baums mit der größten Wahrscheinlichkeit für die beobachteten Messungen der Nahrungsaufnahme, indem die Symbole B (Bisse), F (Nahrungsergänzungen) und A (Artefakte) verwendet werden, um die Ereignisse der Kurve zu interpretieren und eine korrigierte Kurve der Nahrungsaufnahme zu rekonstruieren.

## Revendications

1. Appareil comprenant une balance et un dispositif informatisé connecté à la balance, l'appareil étant configuré pour afficher et représenter une courbe de prise d'aliment pour manger, sur un écran, d'une personne mangeant un repas mesuré à partir d'une vaisselle placée sur la balance communiquant des paramètres alimentaires au dispositif informatisé, **caractérisé en ce que** :
le dispositif informatisé est configuré pour dériver la courbe de prise d'aliment à partir d'un algorithme de grammaire probabiliste hors contexte paramétrique pour l'analyse de prise d'aliment uniquement sur des mesures de poids de repas continues, par le traitement suivant, réalisé en cinq étapes de calcul a.) à e.), par le dispositif informatisé :

a) une phase de prétraitement comprenant un opérateur morphologique d'ouverture avec une taille d'élément de structure adaptative sélectionnée à l'aide de coefficients delta ;
b.) la définition du poids enregistré à chaque temps comme $w(n)$ et la définition des paramètres :

$$d_w(n) = w(n) - w(n-1) \quad \text{et} \quad x(n) = \begin{cases} d \text{ si } d_w(n) < 0 \\ r \text{ si } d_w(n) > 0 \\ e \text{ si } d_w(n) = 0 \end{cases} ;$$

c.) la définition de la grammaire hors contexte :

$$S \rightarrow BS|FS|AS|e \quad (1)$$
$$B \rightarrow D|RD \quad (2)$$
$$F \rightarrow R \quad (3)$$
$$A \rightarrow RSD \quad (4)$$
$$R \rightarrow rr^* \quad (5)$$

(suite)

$$D \rightarrow dd^*\qquad(6)$$

où * est l'opérateur de l'étoile de Kleene,
et les symboles r et d sont les symboles de fin de la grammaire probabiliste hors contexte (PCFG) ;

et où e est le symbole de chaîne vide, les symboles R et D décrivent des segments d'augmentation et de diminution de poids et x est la représentation de chaîne du repas ;
d.) la définition de la probabilité pour les règles de substitution (2), (3) ou (4), correspondant à des événements de Bouchée, Ajout d'aliment ou Artéfact, toutes les autres règles ont une probabilité de 1; et
e.) la sélection de l'arbre d'analyse avec le maximum de vraisemblance pour les mesures de prise d'aliment observées, en utilisant les symboles B (bouchées), F (ajout d'aliment) et A (artéfacts) pour interpréter les événements de la courbe et reconstruire une courbe de prise d'aliment corrigée.

2. Procédé utilisé avec un appareil comprenant une balance et un dispositif informatisé connecté à la balance, le procédé consistant à afficher et à représenter une courbe de prise d'aliment pour manger, sur un écran, d'une personne mangeant un repas mesuré à partir d'une vaisselle placée sur la balance communiquant des paramètres alimentaires au dispositif informatisé, **caractérisé en ce que** :

la courbe de prise d'aliment est dérivée d'un algorithme de grammaire probabiliste hors contexte paramétrique pour l'analyse de prise d'aliment uniquement sur des mesures de poids de repas continues, par le traitement suivant, réalisé en cinq étapes de calcul a.) à e.), par le dispositif informatisé :

a) une phase de prétraitement comprenant un opérateur morphologique d'ouverture avec une taille d'élément de structure adaptative sélectionnée à l'aide de coefficients delta ;
b.) la définition du poids enregistré à chaque temps comme $w(n)$ et la définition des paramètres :

$$d_w(n) = w(n) - w(n-1) \;\texttt{et}\; x(n) = \begin{cases} d \text{ si } d_w(n) < 0 \\ r \text{ si } d_w(n) > 0 \\ e \text{ si } d_w(n) = 0 \end{cases} ;$$

c.) la définition de la grammaire hors contexte :

$$\begin{aligned}
&S \rightarrow BS\,|FS\,|AS\,|e && (1)\\
&B \rightarrow D|\,RD && (2)\\
&F \rightarrow R && (3)\\
&A \rightarrow RSD && (4)\\
&R \rightarrow rr^* && (5)\\
&D \rightarrow dd^* && (6)
\end{aligned}$$

où * est l'opérateur de l'étoile de Kleene,
et les symboles r et d sont les symboles de fin de PCFG ;

et où e est le symbole de chaîne vide, les symboles R et D décrivent des segments d'augmentation et de diminution de poids et x est la représentation de chaîne du repas ;
d.) la définition de la probabilité pour les règles de substitution (2), (3) ou (4), correspondant à des événements de Bouchée, Ajout d'aliment ou Artéfact toutes les autres règles ont une probabilité de 1; et
e.) la sélection de l'arbre d'analyse avec le maximum de vraisemblance pour les mesures de prise d'aliment observées, en utilisant les symboles B (bouchées), F (ajout d'aliment) et A (artéfacts) pour interpréter les événements de la courbe et reconstruire une courbe de prise d'aliment corrigée.

Fig. 1a

Fig. 1b

Fig. 1c

Fig. 2a

Fig. 2b

Fig. 2c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011037513 A

- US 2010236839 A

### Non-patent literature cited in the description

- **CECILIA BERGH ; MONICA CALLMAR ; SOPHIA DANEMAR ; MATS HOLCKE ; SU-¨ SANNE ISBERG ; MICHAEL LEON ; JESSICA LINDGREN ; ASA LUNDQVIST ; MARIA° NIINIMAA ; BARBRO OLOFSSON et al.** Effective treatment of eating disor-~ ders: Results at multiple sites. *Behavioral neuroscience,* 2013, vol. 127 (6), 878
- **CECILIA BERGH ; STALE EKLUND ; MATS ERIKSSON ; GREGER LINDBERG ; PER SODER-STEN.** A new treatment of anorexia nervosa. *The Lancet,* 1996, vol. 348 (9027), 611-612
- **RAFAEL C GONZALEZ ; RICHARD E WOODS.** *Digital image processing,* 2002
- **IOANNIS IOAKIMIDIS ; MODJTABA ZANDIAN ; CECILIA BERGH ; PER SODERSTEN.** A method for the control of eating rate: a potential interven-¨ tion in eating disorders. *Behavior research methods,* 2009, vol. 41 (3), 755-760
- **LOANNIS LOAKIMIDIS ; MODJTABA ZANDIAN ; FLORIAN ULBL ; CARINA ALUND ; CECILIA BERGH ; PER SODERSTEN.** Food intake and chewing in women. *Neurocomputing,* 2012, vol. 84, 31-38
- **HARRY R KISSILEFF ; JOHN THORNTON ; EMIL BECKER.** A quadratic equation adequately describes the cumulative food intake curve in man. *Appetite,* 1982, vol. 3 (3), 255-272

- **VASILEIOS PAPAPANAGIOTOU ; CHRISTOS DIOU ; BILLY LANGLET ; LOANNIS LOAKIMIDIS ; ANASTASIOS DELOPOULOS.** *Automated extraction of food intake indicators from continuous weight measurements,* 2015, vol. 1, 1-2
- **LAURA WEBBER ; DIANA DIVAJEVA ; TIM MARSH ; KLIM MCPHERSON ; MARTIN BROWN ; GAUDEN GALEA ; JOAO BREDA.** The future burden of obesityrelated diseases in the 53 who european-region countries and the impact of effective interventions: a modelling study. *BMJ open,* 2014, vol. 4 (7), e004787
- **RAM WEISS ; SONIA CAPRIO.** The metabolic consequences of childhood obesity. *Best Practice & Research Clinical Endocrinology & Metabolism,* 2005, vol. 19 (3), 405-419
- **MODJTABA ZANDIAN ; IOANNIS IOAKIMIDIS ; CECILIA BERGH ; ULF BRODIN ; PER SODER-STEN.** Decelerated and linear eaters: effect of eating rate on¨ food intake and satiety. *Physiology & behavior,* 2009, vol. 96 (2), 270-275
- **MODJTABA ZANDIAN ; LOANNIS LOAKIMIDIS ; CECILIA BERGH ; PER SODERSTEN.** Linear eaters turned decelerated: Reduction of a risk for¨ disordered eating?. *Physiology & behavior,* 2009, vol. 96 (4), 518-521